# EUROPEAN PATENT APPLICATION

(11) **EP 4 685 194 A1**
(43) Date of publication of application: **28.01.2026**
(21) Application number: 24774823.9
(22) Date of filing: 14.03.2024
(51) Int. Cl.: C08L 101/00, C07C 271/12, C08F 16/28, C08F 20/60

(54) **DEGRADABLE CROSSLINKING AGENT**

(30) Priority: 20.03.2023 JP 2023044476
(71) Applicant: NAGASE CHEMTEX CORPORATION, Osaka-shi, Osaka 550-8668 (JP)
(72) Inventor: KURUSHIMA, Yasunori, Tatsuno-shi, Hyogo 679-4124 (JP)
(74) Representative: Klöckner, Christoph
(86) International application number: PCT/JP2024/010014
(87) International publication number: WO 2024/195685

(57) **Abstract**

The present invention aims to provide a degradable cross-linking agent with an excellent degradation rate. The present invention relates to a degradable cross-linking agent including a compound represented by the following formula (1), the degradable cross-linking agent, when subjected to a reaction with an oxidizing agent after cross-linking, generating at least one water-soluble degradation product including a Z-containing carboxylic acid, a Z-containing alcohol, a Z-containing amine, or a Z-containing thiol and having a water solubility of 30 g/L or more. In formula (1), - n ≥ 1, k ≥ 0, m ≥ 0, p1 ≥ 1, p2 ≥ 1, and p3 ≥ 1; - R¹, R², and R³ each independently represent a single bond or a C1-C500 hydrocarbon group optionally containing a substituent or a heteroatom; - Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group; - each A independently represents a carbonyl group or a single bond; and - each Z independently represents a divalent or higher-valent group containing a siloxane structure or a C1-C500 hydrocarbon group optionally containing a heteroatom.

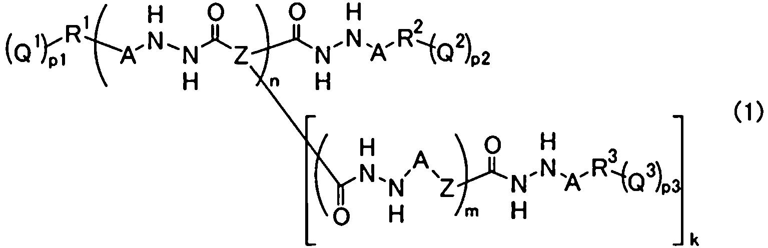

## Description

### TECHNICAL FIELD

The present invention relates to a degradable cross-linking agent.

### BACKGROUND ART

Plastic products are utilized across diverse fields, including daily necessities, automobiles, and electronic devices, owing to their formability, durability, and lightweight properties. However, they present the problem of poor degradability after disposal. In recent years, degradable polymers, which are easily degradable, have been developed for global environmental protection.

Polymers cross-linked with hydrazine derivatives are disclosed as one of degradable polymers (see Patent Literatures 1 and 2). These polymers have a diacyl hydrazine structure and are thus stable in the air, while they can be rapidly degraded when reacted with oxidizing agents such as sodium hypochlorite.

### CITATION LIST

### - Patent Literature

Patent Literature 1: JP 2011-236381 A
Patent Literature 2: WO 2021/131003

### SUMMARY OF INVENTION

### - Technical Problem

The polymers disclosed in Patent Literatures 1 and 2 are not considered to show a sufficient degradation rate when reacted with low concentrations of oxidizing agents. The present invention aims to provide a degradable cross-linking agent with an excellent degradation rate.

### - Solution to Problem

The present inventor has found that a hydrazine derivative containing a highly water-soluble structure can exhibit an enhanced degradation rate. Based on this finding, the present invention has been completed.

Specifically, Embodiment 1 of the present disclosure relates to a degradable cross-linking agent, including a compound represented by the following formula (1): 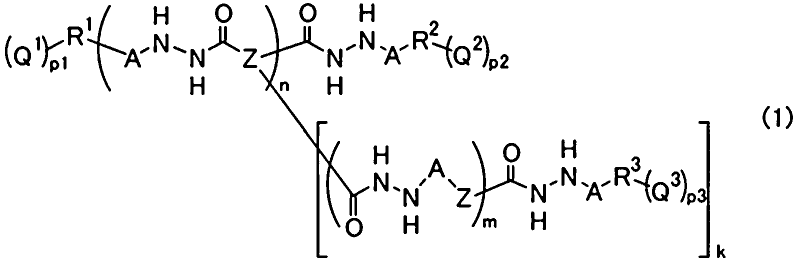 wherein
- n ≥ 1, k ≥ 0, m ≥ 0, p1 ≥ 1, p2 ≥ 1, and p3 ≥ 1;
- R¹, R², and R³ each independently represent a single bond or a C1-C500 hydrocarbon group optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- each A independently represents a carbonyl group or a single bond; and
- each Z independently represents a divalent or higher-valent group containing a siloxane structure or a C1-C500 hydrocarbon group optionally containing a heteroatom,
   the degradable cross-linking agent, when subjected to a reaction with an oxidizing agent after cross-linking, generating at least one water-soluble degradation product including a Z-containing carboxylic acid, a Z-containing alcohol, a Z-containing amine, or a Z-containing thiol and having a water solubility of 30 g/L or more.

Embodiment 2 of the present disclosure is the degradable cross-linking agent according to Embodiment 1,
wherein the water-soluble degradation product is represented by the following formula (2):

Y-Z( Y)ₖ-Y

wherein each Y independently represents a carboxyl group when bound to a carbon atom in Z, or a hydrogen atom when bound to an oxygen atom, a nitrogen atom, or a sulfur atom in Z; and Z and k are each as defined in formula (1).

Embodiment 3 of the present disclosure is the degradable cross-linking agent according to Embodiment 1 or 2,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

Embodiment 4 of the present disclosure is the degradable cross-linking agent according to Embodiment 1 or 2,
wherein p1 = 1, p2 = 1, or p3 = 1.

Embodiment 5 of the present disclosure is the degradable cross-linking agent according to Embodiment 1 or 2,
wherein k ≥ 1.

Embodiment 6 of the present disclosure is the degradable cross-linking agent according to Embodiment 1 or 2,
wherein the degradable cross-linking agent contains, within its molecule, two or more Z groups that differ from each other in structure.

Embodiment 7 of the present disclosure is the degradable cross-linking agent according to Embodiment 1 or 2,
wherein the degradable cross-linking agent, when subjected to the reaction with the oxidizing agent, further generates at least one poorly water-soluble degradation product including a Z-containing carboxylic acid, a Z-containing alcohol, a Z-containing amine, or a Z-containing thiol and having a water solubility of less than 30 g/L.

Embodiment 8 of the present disclosure is the degradable cross-linking agent according to Embodiment 7,
wherein the water-soluble degradation product has a larger average molecular weight than the poorly water-soluble degradation product.

Embodiment 9 of the present disclosure is the degradable cross-linking agent according to Embodiment 7,
wherein a total molar amount of the water-soluble degradation product generated by the reaction with the oxidizing agent is at least 1.3 times a total molar amount of the poorly water-soluble degradation product.

Embodiment 10 of the present disclosure is the degradable cross-linking agent according to Embodiment 1 or 2,
wherein substantially all degradation products containing Z generated by the reaction with the oxidizing agent consist of the water-soluble degradation product.

Embodiment 11 of the present disclosure is the degradable cross-linking agent according to Embodiment 1 or 2,
wherein Z contains a structure represented by the following formula (4):

-(R^{A}O)ₕ- (4)

wherein R^{A} represents a hydrocarbon group optionally having a C1-C5 substituent, and h represents 1 or more.

Embodiment 12 of the present disclosure is the degradable cross-linking agent according to Embodiment 1 or 2,
wherein Z contains at least one hydrophilic functional group selected from the group consisting of a sulfonic acid, a sulfonate, a carboxylic acid, a carboxylate, a hydroxy group, and -NHR^{B} where R^{B} represents a hydrogen atom or a hydrocarbon group optionally having a C1-C5 substituent.

Embodiment 13 of the present disclosure relates to a composition, containing
the degradable cross-linking agent according to Embodiment 1 or 2, and
at least one selected from the group consisting of a curable resin, a polymerization initiator, and a solvent.

Embodiment 14 of the present disclosure relates to a degradable cross-linked product, formed from the composition according to Embodiment 13.

Embodiment 15 of the present disclosure is the degradable cross-linked product according to Embodiment 14, which is degradable with an oxidizing agent.

Embodiment 16 of the present disclosure relates to a method for degrading a degradable cross-linked product, including
bringing the degradable cross-linked product according to Embodiment 14 into contact with an aqueous solution containing an oxidizing agent at 100°C or lower.

### - Advantageous Effects of Invention

The degradable cross-linking agent of the present invention excels in degradation efficiency.

### DESCRIPTION OF EMBODIMENTS

### <<Degradable cross-linking agent>>

The degradable cross-linking agent of the present invention includes a compound represented by the following formula (1): 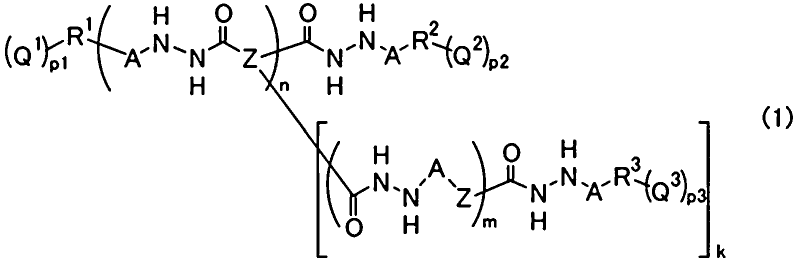 wherein
- n ≥ 1, k ≥ 0, m ≥ 0, p1 ≥ 1, p2 ≥ 1, and p3 ≥ 1;
- R¹, R², and R³ each independently represent a single bond or a C1-C500 hydrocarbon group optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- each A independently represents a carbonyl group or a single bond; and
- each Z independently represents a divalent or higher-valent group containing a siloxane structure or a C1-C500 hydrocarbon group optionally containing a heteroatom. When subjected to a reaction between the cross-linked product and an oxidizing agent after cross-linking, the degradable cross-linking agent generates at least one water-soluble degradation product including a Z-containing carboxylic acid, a Z-containing alcohol, a Z-containing amine, or a Z-containing thiol and having a water solubility of 30 g/L or more.

The degradable cross-linking agent of the present invention is characterized in that a hydrazine structure (-CO-NH-NH-A-) is connected to another hydrazine structure via a structure containing -Z-, so that a water-soluble degradation product containing Z and having a water solubility of 30 g/L or more can be generated by a reaction between the cross-linked product and an oxidizing agent.

It should be noted that although the term "cross-linking agent" may narrowly refer to a chemical substance capable of forming a chemical bond between polymers or within a polymer, the term as used herein refers not only to a chemical substance capable of forming a chemical bond between polymers or within a polymer but also to a chemical substance capable of forming a chemical bond between the molecules of the chemical substance itself.

### <n, m, and k in formula (1)>

In formula (1), n is 1 or more. The upper limit of n is not limited and may be 50 or less. More preferably, n is 1 to 5. If n is more than 50, the solvent solubility tends to decrease. In formula (1), m is 0 or more. The upper limit of m is not limited and may be 30 or less. More preferably, m is 1 to 5. If m is more than 30, the solvent solubility tends to decrease.

In formula (1), k is 0 or more, preferably 1 or more, more preferably 2 or more. When k is 1 or more, the degradable cross-linking agent of the present invention can facilitate three-dimensional cross-linking, resulting in cross-linked products with improved strength and reliability. Additionally, a higher degradation rate can also be obtained. The upper limit of k is not limited and may be 200 or less. If k is more than 200, the solvent solubility tends to decrease.

### <R¹, R², and R³ in formula (1)>

In formula (1), R¹, R², and R³ each independently represent a single bond or a C1-C500 hydrocarbon group optionally containing a substituent or a heteroatom. For the hydrocarbon group, the number of carbon atoms is 1 to 500. In view of the degradation rate, the number of carbon atoms is preferably 1 to 100, more preferably 1 to 10. Examples of the substituent include an alkoxy group, a phenoxy group, a halogen atom, a tertiary amino group, and a sulfo group.

The hydrocarbon group has a saturated or unsaturated hydrocarbon group as its main backbone, which may have a branched or linear structure. Moreover, the backbone formed of the hydrocarbon group may have a cyclic structure. In the hydrocarbon group containing a heteroatom, examples of the heteroatom include N, S, and O. The hydrocarbon group may contain two or more such heteroatoms. The heteroatom may be present on the main chain or a side chain of the hydrocarbon group. Examples of the heteroatom-containing structure include an ether bond, a urethane bond, a urea bond, -NH- in which the hydrogen atom may be substituted, a silicone bond, an ester bond, a thioether bond, and a carbonate bond. The methylene groups other than those at the terminals in the hydrocarbon group may be replaced by one to four heteroatoms selected from N, S, O, and the like, or by arylene or heteroarylene groups. The hydrogen atoms in the hydrocarbon group may be replaced by cyano, nitro, halogen, or phenyl.

Examples of the hydrocarbon group include linear hydrocarbons such as methylene, ethylene, propylene, butylene, pentylene, hexylene, heptylene, octylene, and nonylene; branched hydrocarbons such as isopropylene, isobutylene, 2,2-dimethylpropylene, and 2-ethyl-2-methylpropylene; cyclic hydrocarbons such as cyclohexylene, cyclopentylene, norbomenylene, phenylene, and naphthylene; and PEG chains, as well as trivalent or tetravalent groups corresponding to the foregoing groups.

### <p1, p2, and p3 in formula (1)>

In formula (1), p1, p2, and p3 each represent 1 or more, and are each independently preferably 1 to 4, more preferably 1 to 2. When p1 ≥ 2, p2 ≥ 2, or p3 ≥ 2, the degradable cross-linking agent of the present invention can facilitate three-dimensional cross-linking, resulting in cross-linked products with improved strength and reliability. When p1 ≥ 2, p2 ≥ 2, or p3 ≥ 2, and the plurality of reactive functional groups as Q¹ are different from each other, or the plurality of reactive functional groups as Q² are different from each other, or the plurality of reactive functional groups as Q³ are different from each other, the cross-linking agent allows for flexible selection of various curable resins or cross-linking processes. Moreover, when only a specific reactive functional group is cross-linked, the reactive functional groups not involved in the cross-linking reaction can contribute to improving other physical properties such as adhesiveness or solubility. When p1 = 1, p2 = 1, or p3 = 1, the degradation rate can be further enhanced.

### <Q¹, Q², and Q³ in formula (1)>

In formula (1), Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group. The type of reactive functional group may be selected optimally depending on the application or cross-linking process.

### <Z in formula (1)>

In formula (1), each Z independently represents a divalent or higher-valent group containing a siloxane structure or a C1-C500 hydrocarbon group optionally containing a heteroatom.

When Z is a divalent or higher-valent group containing a siloxane structure, the group containing a siloxane structure contains a -Si-O-bond as a main backbone. The main backbone may be linear, branched, or cyclic. The number of silicon atoms in the group containing a siloxane structure is preferably 2 to 400, more preferably 4 to 200, still more preferably 8 to 150.

In the siloxane structure, the hydrogen atom bound to a silicon atom may be substituted. Specific examples of such substituents include an alkyl group, an alkoxy group, a phenoxy group, a halogen atom, an amino group, a sulfo group, a cyano group, and a nitro group.

Specific examples of the group containing a siloxane structure include dimethyl silicone, diethyl silicone, ethyl methyl silicone, and polymethyl silsesquioxane, as well as modified silicones obtained by modifying a hydrocarbon group at the terminal and/or on a side chain of any of the foregoing groups to contain a heteroatom such as N, S, O, or P.

When Z is a C1-C500 hydrocarbon group optionally containing a heteroatom, the number of carbon atoms thereof is more preferably 2 to 200, still more preferably 4 to 30, in view of the degradation rate and cross-link density. The hydrocarbon group has a saturated or unsaturated hydrocarbon group as a main backbone and may have a branched or linear structure. Moreover, the backbone formed of the hydrocarbon group may have a cyclic structure. The hydrocarbon group may contain a heteroatom such as N, S, or O. The heteroatom may be present in the main chain or a side chain of the hydrocarbon group. Examples of the heteroatom-containing structure include an ether bond, a urethane bond, a urea bond, -NH- in which the hydrogen atom may be substituted, a silicone bond, an ester bond, a thioether bond, and a carbonate bond. The methylene groups other than those at the terminals in the hydrocarbon group may be replaced by one to four heteroatoms selected from N, S, O, and the like, or by arylene or heteroarylene groups. The hydrogen atoms in the hydrocarbon group may be replaced by hydroxy, cyano, amino, nitro, halogen, or phenyl.

Examples of the hydrocarbon group include linear hydrocarbons such as methylene, ethylene, propylene, butylene, pentylene, hexylene, heptylene, octylene, and nonylene; branched hydrocarbons such as isopropylene, isobutylene, 2,2-dimethylpropylene, and 2-ethyl-2-methylpropylene; cyclic hydrocarbons such as cyclohexylene, cyclopentylene, norbomenylene, phenylene, naphthylene, and pyridylene; and PEG chains, as well as trivalent or tetravalent groups corresponding to the foregoing groups.

Z preferably contains a structure represented by the following formula (4):

-(R^{A}O)ₕ- (4)

wherein R^{A} represents a hydrocarbon optionally having a C1-C5 substituent, and h represents 1 or more. Specific examples of the hydrocarbon optionally having a C1-C5 substituent include linear hydrocarbons such as methylene, ethylene, propylene, butylene, pentylene, hexylene, heptylene, octylene, and nonylene; branched hydrocarbons such as isopropylene, isobutylene, 2,2-dimethylpropylene, and 2-ethyl-2-methylpropylene; and cyclic hydrocarbons such as cyclohexylene, cyclopentylene, norbomenylene, phenylene, naphthylene, and pyridylene. In formula (4), h is 1 or more, preferably 2 to 60, more preferably 3 to 15. When the hydrocarbon R^{A} has a substituent, examples of the substituent include an alkoxy group, a phenoxy group, a halogen atom, a tertiary amino group, and a sulfo group. Z containing a structure represented by formula (4) can facilitate dissolution of the degradation product in a solution containing an oxidizing agent, thereby improving the degradation efficiency of the cross-linked product.

The hydrocarbon group as Z also preferably contains a hydrophilic functional group. Examples of the hydrophilic functional group include a sulfonic acid, a sulfonate, a carboxylic acid, a carboxylate, a hydroxy group, and -NHR^{B} where R^{B} represents a hydrogen atom or a hydrocarbon optionally having a C1-C5 substituent, with a sulfonate or a carboxylic acid being preferred. The hydrocarbon group containing a hydrophilic functional group can facilitate dissolution of the degradation product in a solution containing an oxidizing agent, thereby improving the degradation efficiency of the cross-linked product.

When the degradable cross-linking agent contains, within its molecule, two or more Z groups that differ from each other in structure, the degradability can be controlled by selecting a combination of Z types. Examples of such combinations of two or more Z groups in terms of the properties of the degradation product include a combination of Z which imparts high water solubility to the degradation product and Z which imparts low water solubility to the degradation product. Examples of combinations in terms of the properties before degradation include a combination of hydrophilic Z and hydrophobic Z, a combination of Z with high polarity and Z with low polarity, and a combination of Z having a flexible structure and Z having a rigid structure.

### <A in formula (1)>

In formula (1), each A independently represents a carbonyl group or a single bond. When A is a single bond, the degradation efficiency with an oxidizing agent tends to improve. When A is a carbonyl group, the degradation product tends to dissolve easily in a solution containing an oxidizing agent.

### <Oxidizing agent>

When the degradable cross-linking agent of the present invention is subjected to a reaction between an oxidizing agent and the cross-linked product, the hydrazine structures (-CO-NH-NH-A-) are degraded to generate a water-soluble degradation product. The oxidizing agent may be any oxidizing agent other than molecular oxygen. Examples include sodium hypochlorite, potassium hypochlorite, calcium hypochlorite, ammonium hypochlorite, hydrogen peroxide, peracetic acid, m-chloroperoxybenzoic acid, peroxybenzoic acid, ammonium hypobromite, calcium hypobromite, potassium hypobromite, sodium hypobromite, and ozone. These may be used alone or in combination of two or more. In particular, a water-soluble salt such as sodium hypochlorite or sodium hypobromite or ozone water is preferred. These oxidizing agents may each be used in the form of an alkaline aqueous solution such as a sodium hydroxide aqueous solution, a solution in an organic solvent such as ethanol, methanol, tetrahydrofuran, or acetonitrile, or a solution in a solvent mixture of water and the organic solvent.

The oxidizing agent and the cross-linked product may be reacted under any condition. The temperature is preferably 0°C to 100°C, more preferably 15°C to 50°C. The duration is preferably 30 minutes or shorter, more preferably 10 minutes or shorter. If necessary, the cross-linked product may be shaken or stirred during the reaction with the oxidizing agent.

### <Degradation product>

When the degradable cross-linking agent is reacted with a curable resin and the resulting cross-linked product is reacted with an oxidizing agent, at least one degradation product represented by the following formula (2) is generated:

Y-Z(Y)ₖ-Y

wherein each Y independently represents a carboxyl group when bound to a carbon atom in Z, or a hydrogen atom when bound to an oxygen atom, a nitrogen atom, or a sulfur atom in Z; and Z and k are each as defined in formula (1). The degradation product may contain two or more groups selected from a carboxyl group, a hydroxy group, an amino group, or a thiol group.

Examples of the degradation product represented by formula (2) include a degradation product represented by the following formula (2-1) in which all Y groups are carboxyl groups and a degradation product represented by the following formula (2-2) in which all Y groups are hydrogen atoms.

Formula (2-1): HO-CO-Z(COOH)ₖ-COOH

In formula (2-1), Z and k are each as defined in formula (1).

Formula (2-2): H-Z(H)ₖ-H

In formula (2-2), H is bound to an oxygen atom, a nitrogen atom, or a sulfur atom in Z, and Z and k are each as defined in formula (1).

Examples of the degradation product represented by formula (2) also include degradation products represented by the following formula (2-3), formula (2-4), and formula (2-5) in which some of the Y groups are carboxyl group(s) and the others are hydrogen atom(s).

Formula (2-3): HO-CO-Z(H)ₖ-H

In formula (2-3), H is bound to an oxygen atom, a nitrogen atom, or a sulfur atom in Z, and Z and k are each as defined in formula (1).

Formula (2-4): HO-CO-Z(H)ₖ-COOH

In formula (2-4), H is bound to an oxygen atom, a nitrogen atom, or a sulfur atom in Z, and Z and k are each as defined in formula (1).

Formula (2-5): HO-CO-Z(COOH)ₖ-H

In formula (2-5), H is bound to an oxygen atom, a nitrogen atom, or a sulfur atom in Z, and Z and k are each as defined in formula (1).

When the hydrazine structure (-CO-NH-NH-A-) is bound to a hydrocarbon group in Z, the degradation product is a carboxylic acid represented by formula (2-1). Specific examples of the carboxylic acid represented by formula (2-1) include citric acid, succinic acid, malonic acid, adipic acid, phthalic acid, 5-sulfoisophthalic acid, trimellitic acid, and polyacrylic acid.

When the hydrazine structure (-CO-NH-NH-A-) is bound to an oxygen atom, nitrogen atom, or sulfur atom in Z, the degradation product is an alcohol, amine, or thiol represented by formula (2-2). Specific examples of alcohols represented by formula (2-2) include ethylene glycol, diethylene glycol, triethylene glycol, pentaethylene glycol, hexanediol, pentitol, pentaerythritol, polyethylene glycol, poly(vinyl alcohol), ascorbic acid, resorcinol, phenol novolac, and tannic acid. Specific examples of amines represented by formula (2-2) include hexamethylenediamine, pentamethylenediamine, isophoronediamine, toluenediamine, and diaminodiphenylmethane. Specific examples of thiols represented by formula (2-2) include pentaerythritol tetrakis(3-mercaptobutyrate), trimethylolpropane, tris(3-mercaptobutyrate), 1,4-bis(3-mercaptobutyryloxy)butane, and triazine thiol. Specific examples of amino alcohols represented by formula (2-2) include 2-amino-2-methyl-1-propanol, 2-(methylamino)ethanol, valinol, and prolinol. Specific examples of mercapto alcohols represented by formula (2-2) include thioglycol and 1-thioglycerol.

Examples of the degradation product represented by formula (2-3) include glycolic acid, lactic acid, 6-hydroxyhexanoic acid, salicylic acid, gallic acid, glycine, 4-mercaptobenzoic acid, cysteine, serine, and 5-aminosalicylic acid. Examples of the degradation product represented by formula (2-4) include malic acid, glutamic acid, aspartic acid, and methylenedisalicylic acid. Examples of the degradation product represented by formula (2-5) include citric acid, N-(2-hydroxyethyl)ethylenediamine-N,N',N'-triacetic acid, and polyacrylic acid having a hydroxy group at the terminal. Examples of other degradation products represented by formula (2) include acrylic acid-vinyl alcohol copolymers and acrylic acid-vinyl phenol copolymers.

When Z in formula (1) contains the structure represented by formula (4), the degradation product is preferably an alcohol, amine, or thiol. This is because, since the structure represented by formula (4) tends to have a relatively slow dissolution rate in water, the degradation product containing the structure represented by formula (4) and a carboxylic acid may have an insufficient dissolution rate in water.

The degradation products includes at least one water-soluble degradation product having a water solubility of 30 g/L or more. The water solubility of the water-soluble degradation product is preferably 50 g/L or more, more preferably 60 g/L or more. The water solubility is measured in water having a temperature of 20°C. The presence of the water-soluble degradation product having a water solubility of 30 g/L or more improves the degradation efficiency of the cross-linked product. This is presumably because the degradation product dissolves easily in a solution containing an oxidizing agent, which can prevent blockage of the reaction between the oxidizing agent and the cross-linking agent due to deposition of the degradation product, thereby promoting penetration of the solution containing the oxidizing agent into the cross-linked product. However, the present invention is not limited to the above mechanism.

The degradation products may include at least one poorly water-soluble degradation product including a Z-containing carboxylic acid, a Z-containing alcohol, a Z-containing amine, or a Z-containing thiol and having a water solubility of less than 30 g/L. Here, the term "poorly water-soluble degradation product" encompasses a substance having low water solubility as well as a substance that is not dissolved in water at all. Specific examples of the poorly water-soluble degradation product include trimesic acid, decanediol, toluenediamine, and 1,2-ethanedithiol.

The water-soluble degradation product containing Z preferably has a larger average molecular weight than the poorly water-soluble degradation product containing Z. The average molecular weight of the water-soluble degradation product is more preferably at least 1.2 times, still more preferably at least 1.5 times, the average molecular weight of the poorly water-soluble degradation product. When the average molecular weights of the water-soluble degradation product and the poorly water-soluble degradation product are within the above range, the degradation rate tends to increase.

The total molar amount of the water-soluble degradation product generated by the reaction between the cross-linked product and the oxidizing agent is preferably at least 1.3 times, more preferably at least 1.5 times, still more preferably at least 2.0 times, the total molar amount of the poorly water-soluble degradation product. Particularly preferably, substantially all degradation products containing Z generated by the reaction between the cross-linked product and the oxidizing agent consist of the water-soluble degradation product to improve the degradation efficiency of the cross-linked product.

### <Molecular weight>

The molecular weight of the degradable cross-linking agent is not limited, but is preferably 20000 or less, more preferably 400 to 2000, still more preferably 700 to 2000. When the molecular weight is within such a range, the balance between degradability and cross-link density tends to improve. If the molecular weight is more than 20000, the solvent solubility tends to decrease.

### <Method for synthesizing degradable cross-linking agent>

The degradable cross-linking agent may be synthesized by any method. Examples of such methods include syntheses by a dehydration condensation reaction between a hydrazide compound having a reactive functional group and a Z-containing carboxylic acid, a nucleophilic addition reaction of a Z-containing hydrazide compound to an isocyanate having a reactive functional group, an addition reaction between an acid anhydride having a reactive functional group and a Z-containing hydrazide compound, a nucleophilic substitution reaction of a hydrazide compound having a reactive functional group to a Z-containing acid halide, and a polyaddition reaction between hydrazine and a carbonate.

### <Composition>

The composition according to the present invention contains the above-described degradable cross-linking agent and at least one selected from the group consisting of a curable resin, a polymerization initiator, and a solvent. The amount of the degradable cross-linking agent in the composition is preferably 0.1 to 99% by weight, more preferably 1 to 50% by weight.

### <Curable resin>

The curable resin may be any curable resin having a structure that can react with a reactive functional group in the degradable cross-linking agent for cross-linking. Examples of the curable resin include acrylic resins, phenolic resins, epoxy resins, melamine resins, urea resins, unsaturated polyester resins, alkyd resins, silicone resins, isocyanate compounds, and polyimides. The curable resin may be a thermosetting resin or a photocurable resin. The amount of the curable resin in the composition is preferably 0.1 to 95% by weight, more preferably 1 to 50% by weight.

### <Polymerization initiator>

The polymerization initiator may be any compound that can catalyze the polymerization of the curable resin and may be either a thermal polymerization initiator or a photopolymerization initiator. Examples of the polymerization initiator include radical generators such as alkylphenone compounds, benzoin compounds, benzophenone compounds, oxime ester compounds, and phosphine compounds; base generators such as oxime ester compounds, ammonium compounds, benzoin compounds, dimethoxybenzyl urethane compounds, and o-nitrobenzyl urethane compounds; acid generators such as onium salts, halogen-containing compounds, diazomethane compounds, sulfone compounds, and sulfonate compounds; tin compounds such as dibutyltin dilaurate and dibutyltin diacetate; bismuth compounds such as bismuth 2-ethylhexanoate; titanium compounds such as tetraoctyl titanate and titanium ethyl acetoacetate; zirconium compounds such as zirconium monoacetylacetate and zirconium tetraacetylacetate; amines such as triethylenediamine and 1,4-diazabicyclo[2,2,2]octane (DABCO); platinum compounds such as chloroplatinic acid and alkenylsiloxane platinum complexes; iron complexes; and cobalt complexes. The amount of the polymerization initiator in the composition is preferably 0.1 to 10 parts by weight, more preferably 1 to 5 parts by weight, per 100 parts by weight of the curable resin.

### <Solvent>

Examples of the solvent include carbon atom-containing organic solvents such as ether solvents, amide solvents, hydrocarbon solvents, alcohol solvents, ester solvents, aldehyde solvents, ketone solvents, and solvents containing carbon atoms and heteroatoms, as well as water.

Examples of the ether solvents include anisole, 4-methylanisole, diisopropyl ether, diethyl ether, dibutyl ether, tetrahydrofuran, dimethoxyethane, cyclopentyl methyl ether, and tert-butyl methyl ether. Examples of the amide solvents include dimethylformamide, dimethylacetamide, and N-methylpyrrolidone. Examples of the hydrocarbon solvents include aliphatic hydrocarbon solvents such as pentane, hexane, heptane, octane, nonane, decane, undecane, dodecane, and cyclohexane, and aromatic hydrocarbon solvents such as benzene, toluene, xylene, mesitylene, ethylbenzene, tert-butylbenzene, trifluoromethylbenzene, nitrobenzene, and chlorobenzene. Examples of the alcohol solvents include ethanol, propanol, butanol, ethylene glycol, and propylene glycol monomethyl ether. Examples of the ester solvents include ethyl acetate, butyl acetate, and propylene glycol monomethyl ether acetate. Examples of the aldehyde solvents include formaldehyde and acetaldehyde. Examples of the ketone solvents include acetone and methyl ethyl ketone. Examples of the solvents containing carbon atoms and heteroatoms include acetonitrile and dimethyl sulfoxide. The amount of the solvent in the composition is preferably 0.1 to 99% by weight, more preferably 5 to 80% by weight.

### <Degradable cross-linked product>

The degradable cross-linked product according to the present invention can be obtained by cross-linking a composition that contains the degradable cross-linking agent and at least one selected from the group consisting of a curable resin, a polymerization initiator, and a solvent. The degradable cross-linked product may be produced from the composition by any method that can promote the polymerization and cross-linking of the curable resin. A person skilled in the art can appropriately select the conditions for light irradiation, heating, or other processes depending on the types of the degradable cross-linking agent, the curable resin, and the polymerization initiator contained in the composition. For example, a coating film including the degradable cross-linked product can be formed by applying the composition onto a substrate to form a coating film, and subjecting the coating film to light irradiation or heating. Moreover, a three-dimensional formed body of the degradable cross-linked product can be produced by injection molding, compression molding, transfer molding, 3D printing, photolithography, or other methods.

Moreover, a coating film including the degradable cross-linked product can be formed by applying the composition onto a substrate to form a coating film, and subjecting the coating film to light irradiation or heating. The material or form of the substrate is not limited and may be a resin, an inorganic material, paper, or fabric. Examples of the resin include polyesters such as polyethylene terephthalate, polyethylene naphthalate, polylactic acid, polyhydroxy butyric acid, and polybutylene succinate; polyolefins such as polyethylene, polypropylene, and polymethylpentene; cycloolefins, polystyrenes, polytetrafluoroethylene, PMMA, polyamides such as nylon 6 and nylon 66, polycarbonates, poly(vinyl acetate), poly(vinyl alcohol), polyimides, ABS resins, cellulose, cellulose acetate, fibroin, and keratin. Examples of the inorganic material include glass, metals such as Ni, Cu, Cr, Fe, and Si, and oxides or composite materials thereof.

Examples of the method for applying the composition onto the substrate include bar coating, spin coating, spray coating, dip coating, nozzle coating, gravure coating, reverse roll coating, die coating, air doctor coating, blade coating, rod coating, curtain coating, knife coating, transfer roll coating, squeeze coating, impregnation coating, kiss coating, calender coating, and extrusion coating.

The composition applied on the substrate can be cured under any condition. The conditions for curing by light irradiation may include a light irradiation dose of 100 to 2000 mJ/cm². Curing by heating may be performed preferably at a heating temperature of 40°C to 300°C, more preferably 80°C to 120°C. Moreover, the duration of heating is preferably 0.5 to 180 minutes, more preferably 0.5 to 10 minutes.

The thickness of the cured coating film is not limited, but is preferably 0.01 to 900 µm, more preferably 0.05 to 200 µm. The coating film having a thickness within the above range can maintain the strength and adhesion to the substrate.

### <Method for degrading degradable cross-linked product>

The method for degrading a degradable cross-linked product according to the present invention includes bringing the degradable cross-linked product into contact with an aqueous solution containing an oxidizing agent at 100°C or lower.

The oxidizing agent may be any oxidizing agent other than molecular oxygen. Examples include sodium hypochlorite, potassium hypochlorite, calcium hypochlorite, ammonium hypochlorite, hydrogen peroxide, peracetic acid, m-chloroperoxybenzoic acid, peroxybenzoic acid, ammonium hypobromite, calcium hypobromite, potassium hypobromite, sodium hypobromite, and ozone. These may be used alone or in combination of two or more. In particular, a water-soluble salt such as sodium hypochlorite or sodium hypobromite or ozone water is preferred. The solvent for dissolving the oxidizing agent may be an alkali water such as a sodium hydroxide aqueous solution. The solvent may also be a solvent mixture of water and an organic solvent such as ethanol, methanol, tetrahydrofuran, or acetonitrile. The oxidizing agent concentration in the aqueous solution is preferably 0.001 to 50% by weight, more preferably 0.01 to 20% by weight, still more preferably 0.1 to 10% by weight, particularly preferably 0.3 to 5% by weight.

The conditions for bringing the degradable cross-linked product into contact with the aqueous solution containing the oxidizing agent include a temperature of 100°C or lower, preferably of 15°C to 50°C. The conditions preferably include a duration of 30 minutes or shorter, more preferably 10 minutes or shorter. If necessary, the cross-linked product may be shaken or stirred during the reaction with the oxidizing agent. Non-limiting specific examples of the method for bringing the degradable cross-linked product into contact with the aqueous solution containing the oxidizing agent include immersing the degradable cross-linked product in the aqueous solution containing the oxidizing agent, or spraying or adding dropwise the aqueous solution containing the oxidizing agent onto the degradable cross-linked product.

### <Application of degradable cross-linking agent>

Since the degradable cross-linking agent of the present invention has an excellent degradation rate, it can be suitably used in various applications requiring high productivity. Examples of such applications include paints, adhesives, pressure-sensitive adhesives, water-absorbent resins, resins for 3D printing, photoresists, release agents, cell culture media, immobilization materials for affected areas, and imprint molded articles.

### EXAMPLES

The present invention is described below with reference to examples, but the present invention is not limited thereto. Hereinafter, the units "part(s)" and "%" refer to "part(s) by weight" and "% by weight", respectively, unless otherwise specified.

### (Example 1)

An amount of 1.0 g of triethylene glycol bis(succinimidyl carbonate) was dissolved in 9.2 g of tetrahydrofuran in a 20 mL round-bottom flask. The solution was adjusted to a temperature of 0°C, and 0.36 g of methacrylic acid hydrazide was added to the resulting solution. The mixture was stirred at room temperature for three hours and then concentrated with an evaporator. To the residue was added 53 g of diethyl ether. The resulting precipitate was dried in a vacuum dryer at 40°C to obtain a compound of formula (A1) at a yield of 90%. 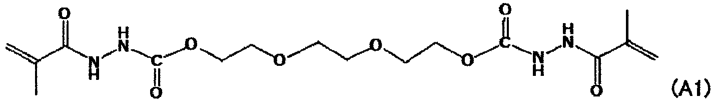 1H-NMR (DMSO, δ ppm) 1.86 (6H, S, CH₃), 3.55 to 3.60 (8H, m, CH₂OCH₂), 4.12 (4H, s, COOCH₂), 5.44 (2H, s, C=CH₂), 5.71 (2H, s, C=CH₂), 9.11 (2H, S, NHNH), 9.77 (2H, S, NHNH)

Upon adding a small amount of a 12% sodium hypochlorite aqueous solution to the obtained compound of formula (A1), foaming occurred. 1H-NMR analysis confirmed the generation of triethylene glycol as a degradation product. One gram of triethylene glycol was soluble in 10 mL of ion exchange water having a temperature of 20°C, exhibiting water solubility.

An amount of 0.3 g of the compound of formula (A1), 0.1 g of acryloylmorpholine (ACMO available from KJ Chemicals Corporation), and 12 mg of a photopolymerization initiator (Omnirad 907 available from IGM Resins) were dissolved in 1 g of N-methylpyrrolidone to prepare a composition for producing a degradable formed product. The composition was applied onto a glass plate to give a dry film thickness of 10 µm. After drying the composition with a fan dryer at 150°C for five minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate and dissolved in the solution within 10 minutes. 1H-NMR analysis confirmed the presence of triethylene glycol as a degradation product.

### (Example 2)

An amount of 11.1 g of poly(acrylic acid hydrazide) having a molecular weight of about 1000 and 138 g of dimethyl sulfoxide were mixed in a 300 mL round-bottom flask, and then 5.0 g of 2-isocyanatoethyl methacrylate was added dropwise at room temperature. After stirring for four hours, 10.6 g of succinic anhydride was added, followed by further stirring for two hours. The reaction liquid was added dropwise to 1651 g of methyl isobutyl ketone. The resulting solid was dried in a vacuum dryer at 50°C to obtain a compound having a structure of formula (A2) at a yield of 60%. 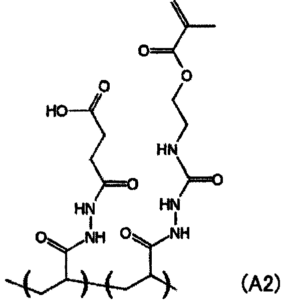 1H-NMR (DMSO, δ ppm) 1.55 (20H, br, main chain CH₂), 2.11 (10H, br, main chain CH), 1.88 (9H, s, CH₃), 2.46 (28H, br, COCH₂CH₂CO), 3.47 to 3.51 (6H, m, NCH₂), 4.04 to 4.08 (6H, m, OCH₂), 5.67 (3H, d, C=CH₂), 6.06 (3H, d, C=CH₂), 6.43 to 6.52 (3H, m, CONHC), 9.82 (20H, br, NHNH), 12.12 (7H, br, COOH)

Upon adding a small amount of a 12% sodium hypochlorite aqueous solution to the obtained compound of formula (A2), foaming occurred. 1H-NMR analysis confirmed the generation of polyacrylic acid as a degradation product. An amount of 0.5 g of polyacrylic acid was soluble in 10 mL of ion exchange water having a temperature of 20°C, exhibiting water solubility.

An amount of 0.3 g of the compound of formula (A2) and 12 mg of a photopolymerization initiator (Omnirad 907 available from IGM Resins) were dissolved in 1.5 g of a solvent mixture of N-methylpyrrolidone and dimethyl sulfoxide (weight ratio = 1:1) to prepare a composition for producing a degradable formed product. The composition was applied onto a glass plate to give a dry film thickness of 10 µm. After drying the composition with a fan dryer at 150°C for five minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate and dissolved in the solution within one minute.

An amount of 0.3 g of the compound of formula (A2), 0.1 g of acryloylmorpholine (ACMO available from KJ Chemicals Corporation), and 12 mg of a photopolymerization initiator (Omnirad 907 available from IGM Resins) were dissolved in 1.5 g of a solvent mixture of N-methylpyrrolidone and dimethyl sulfoxide (weight ratio = 1:1) to prepare a composition for producing a degradable formed product. The composition was applied onto a glass plate to give a dry film thickness of 10 µm. After drying the composition with a fan dryer at 150°C for five minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate and dissolved in the solution within one minute. 1H-NMR analysis confirmed the presence of polyacrylic acid as a degradation product.

### (Example 3)

An amount of 10.6 g of tetrahydrofuran, 0.48 g of hexyl dicarbazate, and 2.1 g of pure water were mixed in a 30 mL round-bottom flask, and then 0.82 g of N-(allyloxycarbonyloxy)succinimide was added under ice cooling. After stirring for four hours, the solvent was removed with an evaporator, and then 30 g of chloroform and 36 g of pure water were added. After removing the aqueous layer by a liquid separation process, the organic layer was concentrated with an evaporator to precipitate a white solid. The resulting solid was washed with 34 g of pure water and dried in a vacuum dryer at 40°C to obtain a compound of formula (A3) at a yield of 22%. 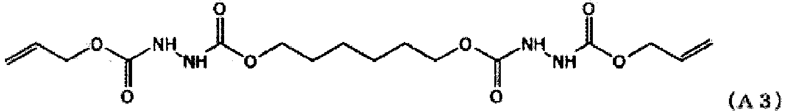 1H-NMR (DMSO, δ ppm) 1.32 (4H, br, CH₂), 1.55 (4H, br, CH₂), 3.99 (4H, t, OCH₂), 4.52 (4H, dt, OCH₂), 5.20 (2H, d, C=CH₂), 5.30 (2H, d, C=CH₂), 5.85 to 5.95 (2H, m, CH), 9.03 (2H, s, NHNH), 9.11 (2H, s, NHNH)

Upon adding a small amount of a 12% sodium hypochlorite aqueous solution to the obtained compound of formula (A3), foaming occurred. 1H-NMR analysis confirmed the generation of 1,6-hexanediol as a degradation product. One gram of 1,6-hexanediol was soluble in 10 mL of ion exchange water having a temperature of 20°C, exhibiting water solubility.

An amount of 0.8 g of the compound of formula (A3), 0.6 g of pentaerythritol tetrakis(3-mercaptobutyrate), 0.7 g of dimethyl sulfoxide, 3.2 g of ethyl acetate, 3.2 g of methyl isobutyl ketone, 33 mg of a photopolymerization initiator (Omnirad 907 available from IGM Resins), and 33 mg of a photopolymerization initiator (Omnirad TPO-H available from IGM Resins) were mixed to prepare a composition for producing a degradable formed product. The composition was applied onto a PET film (Lumirror T60 available from Toray Industries, Inc.) to give a dry film thickness of 10 µm. After drying the composition with a fan dryer at 120°C for five minutes, the dried composition was exposed to ultraviolet rays at 500 mJ/cm² using a UV irradiation device (Unicure UVH-1500M available from USHIO Inc.) to obtain a degradable formed product. The adhesion of the degradable formed product to the PET film was determined by a cross-cut test (JIS K 5600-5-6) and was found to be classified as 0, confirming good adhesion. Moreover, the degradable formed product was insoluble in ion exchange water and acetone, but when it was immersed in a 1% sodium hypochlorite aqueous solution having a temperature of 20°C, foaming occurred, and the degradable formed product was completely removed from the substrate and dissolved in the solution within four minutes. 1H-NMR analysis confirmed the presence of 1,6-hexanediol as a degradation product.

### (Example 4)

An amount of 2.4 g of citric acid trihydrazide was dissolved in 53 g of dimethylformamide in a 100 mL round-bottom flask, and then 6.7 g of N-allyloxycarbonyloxy succinimide was added at 5°C, followed by stirring at room temperature overnight. The reaction liquid was concentrated with an evaporator. Subsequently, 400 g of ethyl acetate was added to the residue, and a precipitate was collected. Then, the precipitate was dried in a vacuum dryer to obtain a compound of formula (A4) as a white solid at a yield of 40%. 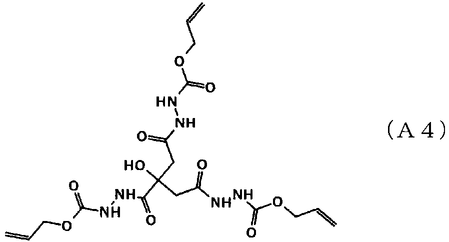 1H-NMR (DMSO, δ ppm) 2.81 (4H, br, C=OCH₂), 4.51 (6H, br, OCH₂), 5.20 (3H, d, C=CH), 5.30 (3H, d, C=CH), 5.74 (1H, br, OH), 5.89 (3H, br, C=CH), 9.13 (3H, br, NH), 9.74 (3H, br, NH)

Upon adding a small amount of a 12% sodium hypochlorite aqueous solution to the obtained compound of formula (A4), foaming occurred. 1H-NMR analysis confirmed the generation of citric acid as a degradation product. Three grams of citric acid was soluble in 10 mL of ion exchange water having a temperature of 20°C, exhibiting water solubility.

An amount of 0.4 g of the compound of formula (A4), 0.34 g of pentaerythritol tetrakis(3-mercaptobutyrate), 3.3 g of dimethyl sulfoxide, 17 mg of a photopolymerization initiator (Omnirad 907 available from IGM Resins), and 17 mg of a photopolymerization initiator (Omnirad TPO-H available from IGM Resins) were mixed to prepare a composition for producing a degradable formed product. The composition was applied onto a glass plate to give a dry film thickness of 10 µm. After drying the composition with a fan dryer at 120°C for 15 minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water and acetone, but when it was immersed in a 1% sodium hypochlorite aqueous solution having a temperature of 20°C, foaming occurred, and the degradable formed product was completely removed from the substrate within two minutes. 1H-NMR analysis confirmed the presence of citric acid in the degradation product.

### (Example 5)

Four grams of methanol and 3.0 g of citric acid trihydrazide were added to and dissolved in 8 g of ion exchange water in a 100 mL round-bottom flask. Subsequently, 8.3 g of 4-hydroxybutyl acrylate was added to the solution, and the mixture was stirred at room temperature for five days. The reaction liquid was added dropwise to 130 g of acetonitrile, and the upper layer was removed. The lower layer was further washed with 60 g of acetonitrile. Thereafter, the residue was dried in a vacuum dryer at 50°C for three hours to obtain a compound of formula (A5) as a viscous liquid at a yield of 55%. 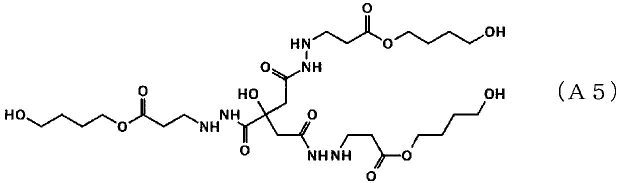 1H-NMR (DMSO, δ ppm) 1.41 to 1.48 (6H, m, CH₂), 1.56 to 1.63 (6H, m, CH₂), 2.38 to 2.51 (10H, m, O=CCH₂), 2.84 to 3.00 (6H, m, NCH₂), 3.40 (6H, t, OCH₂), 4.01 (6H, t, O=COCH₂), 4.41 (3H, br, OH), 4.95 (3H, br, NHNH), 6.02 (1H, br, OH), 9.20 (3H, br, NHNH)

Upon adding a small amount of a 12% sodium hypochlorite aqueous solution to the obtained compound of formula (A5), foaming occurred. 1H-NMR analysis confirmed the generation of citric acid as a degradation product. Three grams of citric acid was soluble in 10 mL of ion exchange water having a temperature of 20°C, exhibiting water solubility.

To 1.0 g of hexamethylene diisocyanate and 3 g of the compound of formula (A5), 100 mg of di-n-butyltin dilaurate was added, and the mixture was stirred at 60°C for 30 minutes to prepare a composition for producing a degradable formed product. The composition was applied onto a glass plate to give a dry film thickness of 10 µm. The composition was then cured with a fan dryer at 90°C for two hours to obtain a degradable formed product. The degradable formed product was insoluble in ion exchange water and acetone, but when it was immersed in a 1% sodium hypochlorite aqueous solution having a temperature of 20°C, foaming occurred, and the degradable formed product was completely removed from the substrate within two minutes. 1H-NMR analysis confirmed the presence of citric acid in the degradation product.

### (Example 6)

An amount of 1.8 g of sodium 5-sulfoisophthalate dihydrazide was dissolved in 40 g of methyl sulfoxide in a 100 mL three-necked flask. Then, 3.4 g of diethyl 2-isocyanatoglutarate was added to the solution, and the mixture was stirred at 80°C overnight. After the reaction mixture was left to cool to room temperature, it was diluted with 100 g of pure water. The aqueous layer was washed three times with 90 g of ethyl acetate and then concentrated. The resulting residue was dried in vacuum. The resulting pale orange solid was transferred to a 30 mL round-bottom flask, and 4.6 g of hydrazine monohydrate was added thereto, followed by stirring at room temperature for four hours. The reaction mixture was added dropwise to 200 g of methanol to precipitate a white solid. The obtained solid was collected by filtration and then dried in vacuum to obtain a compound of formula (A6) at a yield of 93%. 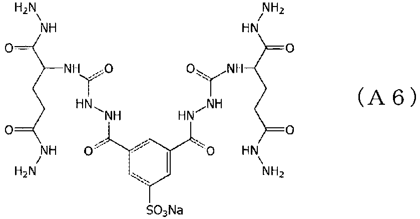 1H-NMR (DMSO, δ ppm) 1.70 to 1.86 (4H, m, CH₂), 1.97 to 2.09 (4H, m, O=CCH₂), 4.13 (2H, q, O=CCH), 4.23 (8H, br, NH₂), 6.72 (2H, br, CNHC), 8.12 (2H, s, NHNH), 8.30 (2H, s, benzene ring), 8.32 (1H, s, benzene ring), 8.98 (2H, s, NNH), 9.15 (2H, br, NNH), 10.41 (2H, br, NHNH)

Upon adding a small amount of a 12% sodium hypochlorite aqueous solution to the obtained compound of formula (A6), foaming occurred. 1H-NMR analysis confirmed the generation of sodium 5-sulfoisophthalate as a degradation product. One gram of sodium 5-sulfoisophthalate was soluble in 10 mL of ion exchange water having a temperature of 20°C, exhibiting water solubility.

An amount of 0.50 g of the compound of formula (A6) was dissolved in 10 g of pure water. Then, 0.73 g of polyglycerol polyglycidyl ether (GEX-521 available from Nagase ChemteX Corporation) and 0.03 g of a leveling agent (BYK-348 available from BYK Japan KK) were added to the solution to prepare a composition for producing a degradable formed product. The composition was applied onto a glass substrate to give a cured film thickness of 10 µm, and then heated at 80°C for three hours to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate within 12 minutes. 1H-NMR analysis confirmed the presence of sodium 5-sulfoisophthalate in the degradation product.

### (Example 7)

An amount of 2.9 g of the compound of formula (A6) was dissolved in 33 g of dimethyl sulfoxide in a 100 mL round-bottom flask, and then 4.1 g of N-allyloxycarbonyloxy succinimide was added to the solution, followed by stirring at room temperature overnight. The reaction liquid was transferred to a separatory funnel and diluted with 105 g of pure water. The resulting aqueous layer was washed three times with 80 g of methylene chloride and then concentrated to obtain a pale orange solid. The solid was washed three times with 150 g of methylene chloride and then dried in vacuum to obtain a compound of formula (A7) at a yield of 83%. 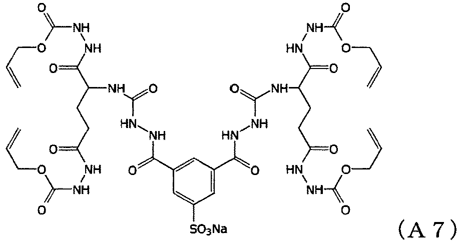 1H-NMR (DMSO, δ ppm) 1.72 to 1.94 (4H, m, CH₂), 2.19 (4H, br, O=CCH₂), 4.26 (2H, q, O=CCH), 4.50 to 4.55 (8H, m, OCH₂), 5.20 (4H, dd, C=CH₂), 5.31 (4H, dd, C=CH₂), 5.87 to 5.95 (2H, m, CH=C), 6.69 (2H, br, CNHC), 8.18 (2H, s, NHNH), 8.28 (2H, s, benzene ring), 8.30 (1H, s, benzene ring), 9.06 (2H, s, NHNH), 9.24 (2H, s, NHNH), 9.69 (2H, s, NHNH), 9.89 (2H, br, NHNH), 10.40 (2H, br, NHNH)

Upon adding a small amount of a 12% sodium hypochlorite aqueous solution to the obtained compound of formula (A7), foaming occurred. 1H-NMR analysis confirmed the generation of sodium 5-sulfoisophthalate and glutamic acid as degradation products. One gram of sodium 5-sulfoisophthalate was soluble in 10 mL of ion exchange water having a temperature of 20°C, exhibiting water solubility, while 0.3 g of glutamic acid was insoluble in 10 mL of ion exchange water having a temperature of 20°C, exhibiting water insolubility.

An amount of 0.5 g of the compound of formula (A7), 0.27 g of pentaerythritol tetrakis(3-mercaptobutyrate), 3.3 g of methanol, 17 mg of a photopolymerization initiator (Omnirad 907 available from IGM Resins), and 17 mg of a photopolymerization initiator (Omnirad TPO-H available from IGM Resins) were mixed to prepare a composition for producing a degradable formed product. The composition was applied onto a PET film (Lumirror T60 available from Toray Industries, Inc.) to give a dry film thickness of 10 µm. After drying the composition with a fan dryer at 120°C for two minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water and acetone, but when it was immersed in a 1% sodium hypochlorite aqueous solution having a temperature of 20°C, foaming occurred, and the degradable formed product was completely removed from the substrate within 15 minutes. 1H-NMR analysis confirmed the presence of sodium 5-sulfoisophthalate in the degradation product.

### (Example 8)

An amount of 5.0 g of succinic acid dihydrazide was dissolved in 30 g of methyl sulfoxide in a 100 mL three-necked flask. Then, 15.7 g of diethyl 2-isocyanatoglutarate was added to the solution, and the mixture was stirred at room temperature for one day. Subsequently, 24.7 g of hydrazine monohydrate was added, and the mixture was further stirred for one day. The reaction liquid was added dropwise to 900 g of methanol to precipitate a solid. The obtained solid was washed with 300 g of methanol and dried in a vacuum dryer at 40°C for three hours. Five grams of the dried solid was transferred to a 100 mL round-bottom flask and dissolved in 23 g of dimethyl sulfoxide. To the solution, 7.3 g of N-allyloxycarbonyloxy succinimide was added, followed by stirring at room temperature for one day. The reaction liquid was added dropwise to 550 g of acetonitrile to precipitate a solid. The solid was washed with 250 g of acetonitrile and dried in a vacuum dryer at 50°C for three hours to obtain 2.6 g of a compound of formula (A8). 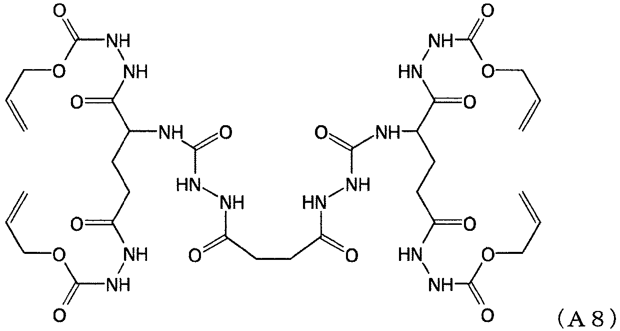 1H-NMR (DMSO, δ ppm) 1.68 to 1.91 (4H, m, CH₂), 2.04 to 2.17 (4H, m, C=OCH₂), 2.37 (4H, br, C=OCH₂), 4.07 to 4.13 (2H, m, C=OCH), 4.51 (8H, br, OCH₂), 5.19 (4H, d, C=CH₂), 5.30 (4H, d, C=CH₂), 5.89 (4H, br, CH=C), 6.52 (2H, br, NC=ONHC), 7.90 (2H, s, NHNH), 9.03 (2H, br, NHNH), 9.19 (2H, br, NHNH), 9.57 (2H, br, NHNH), 9.64 (2H, br, NHNH), 9.81 (2H, br, NHNH)

Upon adding a small amount of a 12% sodium hypochlorite aqueous solution to the obtained compound of formula (A8), foaming occurred. 1H-NMR analysis confirmed the generation of succinic acid and glutamic acid as degradation products. An amount of 0.5 g of succinic acid was soluble in 10 mL of ion exchange water having a temperature of 20°C, exhibiting water solubility, while 0.3 g of glutamic acid was insoluble in 10 mL of ion exchange water having a temperature of 20°C, exhibiting water insolubility.

An amount of 0.5 g of the compound of formula (A8), 0.31 g of pentaerythritol tetrakis(3-mercaptobutyrate), 2.3 g of propylene glycol monomethyl ether, 1.0 g of N-methylpyrrolidone, 17 mg of a photopolymerization initiator (Omnirad 907 available from IGM Resins), and 17 mg of a photopolymerization initiator (Omnirad TPO-H available from IGM Resins) were mixed to prepare a composition for producing a degradable formed product. The composition was applied onto a PET film (Lumirror T60 available from Toray Industries, Inc.) to give a dry film thickness of 10 µm. After drying the composition with a fan dryer at 120°C for five minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water and acetone, but when it was immersed in a 1% sodium hypochlorite aqueous solution having a temperature of 20°C, foaming occurred, and the degradable formed product was completely removed from the substrate within 20 minutes. 1H-NMR analysis confirmed the presence of succinic acid in the degradation product.

### (Example 9)

An amount of 80 g of hexamethylene diisocyanate was provided in a 300 mL round-bottom flask, and a solution of 10 g of trimesic acid trihydrazide in 170 g of dimethyl sulfoxide was added dropwise thereto. The mixture was stirred in a nitrogen atmosphere at room temperature for three hours. The reaction liquid was added dropwise to 550 g of THF to precipitate a solid. The obtained solid was removed, and the filtrate was concentrated and then added dropwise to 1 L of toluene. After removing the upper layer, the lower layer was washed with 100 g of toluene. The resulting lower layer was dried in a vacuum dryer at 20°C for two hours., and then 63 g of dimethyl sulfoxide was added to dissolve the residue. The resulting solution was transferred to a 100 mL round-bottom flask, and 3.9 g of methacrylic acid hydrazide was added thereto. After stirring at room temperature overnight, the formed solid was removed by filtration. The filtrate was added dropwise to 500 g of acetone to precipitate a white solid. The obtained white solid was dried in a vacuum dryer at 20°C for one hour to obtain a compound of formula (A9) at a yield of 22%. 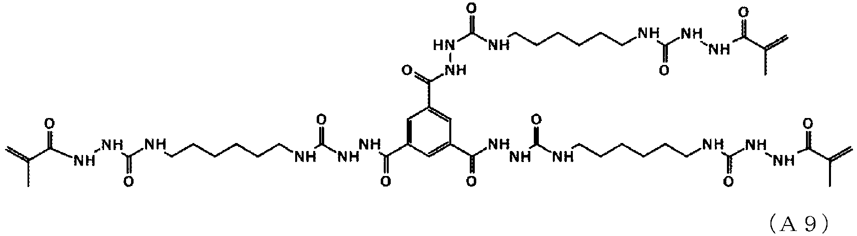 1H-NMR (DMSO, δ ppm) 1.23 to 1.28 (12H, m, CH₂), 1.33 to 1.44 (12H, m, CH₂), 1.86 (9H, s, CH₃), 2.94 to 3.05 (12H, m, NCH₂), 5.39 (3H, br, C=CH₂), 5.74 (3H, br, C=CH₂), 6.32 (3H, br, NH), 6.51 (3H, br, NH), 7.60 (3H, br, NHNH), 7.94 (3H, s, benzene ring), 8.51 (3H, br, NHNH), 9.55 (3H, br, NHNH), 10.23 (3H, br, NHNH)

Upon adding a small amount of a 12% sodium hypochlorite aqueous solution to the obtained compound of formula (A9), foaming occurred. 1H-NMR analysis confirmed the generation of trimesic acid and hexamethylenediamine as degradation products. One gram of hexamethylenediamine was soluble in 10 mL of ion exchange water having a temperature of 20°C, exhibiting water solubility, while 0.3 g of trimesic acid was insoluble in 10 mL of ion exchange water having a temperature of 20°C, exhibiting water insolubility.

An amount of 0.3 g of the compound of formula (A9), 0.1 g of acryloylmorpholine (ACMO available from KJ Chemicals Corporation), and 12 mg of a photopolymerization initiator (Omnirad 907 available from IGM Resins) were dissolved in 2.0 g of dimethyl sulfoxide to prepare a composition for producing a degradable formed product. The composition was applied onto a glass plate to give a dry film thickness of 10 µm. After drying the composition with a fan dryer at 150°C for 10 minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate within 10 minutes. 1H-NMR analysis confirmed the presence of hexamethylenediamine in the degradation product.

### (Example 10)

An amount of 16 g of acetonitrile, 2.5 g of pentaethylene glycol, and 3.2 g of triethylamine were mixed in a 100 mL round-bottom flask. Thereafter, 6.1 g of di(N-succinimide) carbonate was added and the mixture was stirred at room temperature for three hours. Next, 0.92 g of adipic acid dihydrazide was added, and the mixture was stirred for two hours. To the resulting mixture, 16 g of dimethyl sulfoxide was added, and 13 g of the solvent was removed by evaporation with an evaporator. To the thus concentrated reaction liquid, 1.56 g of sodium 5-sulfoisophthalate dihydrazide was added and the mixture was stirred at room temperature overnight. Then, 0.35 g of sodium 5-sulfoisophthalate dihydrazide was added again, and the mixture was stirred at room temperature overnight and then added dropwise to 150 g of acetone. The upper layer was discarded, and the lower layer was washed with 50 g of methanol. The resulting lower layer was dried in a vacuum dryer at 20°C overnight to obtain a compound of formula (A10) at a yield of 83%. 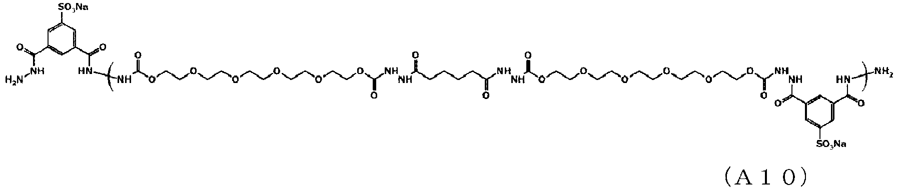 1H-NMR (DMSO, δ ppm) 1.51 (76H, br, CH₂), 2.08 (76H, br, CH₂), 3.42 to 3.64 (608H, m, OCH₂), 4.10 (76H, br, O=COCH₂), 4.16 (76H, br, O=COCH₂), 4.61 (4H, br, NH₃), 8.28 (60H, br, benzene ring), 9.05 (38H, br, NHNH), 9.35 (38H, br, NHNH), 9.58 (38H, br, NHNH), 9.92 (2H, br, NHNH), 10.55 (38H, br, NHNH)

Upon adding a small amount of a 12% sodium hypochlorite aqueous solution to the obtained compound of formula (A10), foaming occurred. 1H-NMR analysis confirmed the generation of adipic acid, 5-sulfoisophthalic acid, and pentaethylene glycol as degradation products. One gram of 5-sulfoisophthalic acid and one gram of pentaethylene glycol were each soluble in 10 mL of ion exchange water having a temperature of 20°C, exhibiting water solubility, while 0.3 g of adipic acid was insoluble in 10 mL of ion exchange water having a temperature of 20°C, exhibiting water insolubility.

An amount of 4.0 g of the compound of formula (A10), 4.0 g of dimethylformamide, and 0.35 g of resorcinol diglycidyl ether (EX-201-IM available from Nagase ChemteX Corporation) were mixed to prepare a composition for producing a degradable formed product. The composition was applied onto a glass substrate to give a cured film thickness of 10 µm, and then heated at 80°C for three hours to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate within 10 minutes. 1H-NMR analysis confirmed the presence of sodium 5-sulfoisophthalate and pentaethylene glycol in the degradation products.

### (Example 11)

An amount of 14.5 g of N-hydroxysuccinimide was dissolved in 377 g of THF in a 1 L round-bottom flask. Then, 9.5 g of isophthalic acid and 24.3 g of 1-ethyl-3-(3-dimethylaminopropyl)carbodiimide hydrochloride were added to the solution, and the mixture was stirred at room temperature overnight. After the resulting mixture was concentrated with an evaporator, 310 g of chloroform was added and the mixture was transferred to a separatory funnel. The organic layer was washed four times with 200 g of ion exchange water. The organic layer was dried over 5.8 g of magnesium sulfate and then concentrated with an evaporator. The concentrated liquid was added dropwise to 150 g of isopropanol to precipitate a solid. The obtained solid was dried in a vacuum dryer at 25°C overnight. An amount of 13.6 g of 6-hydroxyhexanoic acid hydrazide, 18 g of triethylamine, and 40 g of dimethylformamide were mixed in a 100 mL round-bottom flask, and 15.3 g of the dried solid was added thereto. After stirring at room temperature overnight, the reaction liquid was added dropwise to 800 g of acetonitrile to precipitate a white powder. The white powder was collected by filtration, washed with 200 g of acetonitrile, and then dried in a vacuum dryer at 50°C for three hours. Six grams of the dried white powder was transferred to a 100 mL round-bottom flask and dissolved in 36 g of dimethylformamide. Then, 8.7 g of triethylamine and 7.3 g of di(N-succinimidyl)carbonate were added to the solution, and the mixture was stirred at room temperature for 90 minutes. Then, 3.4 g of 2-hydroxyethyl carbazate was added and the mixture was further stirred overnight. The reaction liquid was added dropwise to 800 g of acetonitrile to precipitate a solid. The solid was washed with 200 g of acetonitrile and dried in a vacuum dryer at 50°C for three hours to obtain 6.7 g of a compound of formula (A11). 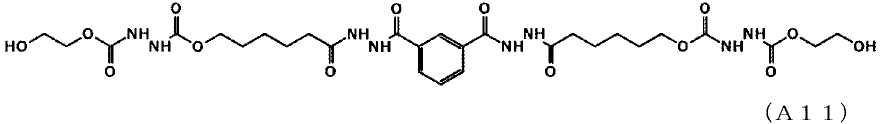 1H-NMR (DMSO, δ ppm) 1.31 to 1.63 (12H, m, CH₂), 2.17 to 2.24 (4H, m, O=CCH₂), 3.55 (4H, q, OCH₂), 3.99 to 4.02 (8H, q, O=COCH₂), 4.77 (2H, br, OH), 7.62 (1H, t, benzene ring), 8.03 (2H, dd, benzene ring), 8.37 (1H, s, benzene ring), 9.00 (2H, br, NHNH), 9.03 (2H, br, NHNH), 9.99 (2H, br, NHNH), 10.40 (2H, br, NHNH)

Upon adding a small amount of a 12% sodium hypochlorite aqueous solution to the obtained compound of formula (A11), foaming occurred. 1H-NMR analysis confirmed the generation of 6-hydroxyhexanoic acid and isophthalic acid as degradation products. An amount of 0.5 g of 6-hydroxyhexanoic acid was soluble in 10 mL of ion exchange water having a temperature of 20°C, exhibiting water solubility, while 0.3 g of isophthalic acid was insoluble in 10 mL of ion exchange water having a temperature of 20°C, exhibiting water insolubility.

An amount of 0.75 g of polymethylene polyphenyl polyisocyanate (Millionate MR-200 available from Tosoh Corporation) and 2.0 g of the compound of formula (A11) were dissolved in 10 mL of dimethylformamide. To the solution, 75 mg of di-n-butyltin dilaurate was added to prepare a composition for producing a degradable formed product. The composition was applied onto a glass plate to give a dry film thickness of 10 µm, and heated with a fan dryer at 90°C for two hours to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate within 20 minutes. 1H-NMR analysis confirmed the presence of 6-hydroxyhexanoic acid in the degradation product.

### (Example 12)

An amount of 14.5 g of N-hydroxysuccinimide was dissolved in 377 g of THF in a 1 L round-bottom flask. Then, 9.5 g of isophthalic acid and 24.3 g of 1-ethyl-3-(3-dimethylaminopropyl)carbodiimide hydrochloride were added to the solution, and the mixture was stirred at room temperature overnight. After the resulting mixture was concentrated with an evaporator, 310 g of chloroform was added and the mixture was transferred to a separatory funnel. The organic layer was washed four times with 200 g of ion exchange water. The organic layer was dried over 5.8 g of magnesium sulfate and then concentrated with an evaporator. The concentrated liquid was added dropwise to 150 g of isopropanol to precipitate a solid. The obtained solid was dried in a vacuum dryer at 25°C overnight. An amount of 13.6 g of 6-hydroxyhexanoic acid hydrazide, 18 g of triethylamine, and 40 g of dimethylformamide were mixed in a 100 mL round-bottom flask, and 15.3 g of the dried solid was added thereto. After stirring at room temperature overnight, the reaction liquid was added dropwise to 800 g of acetonitrile to precipitate a white powder. The white powder was collected by filtration, washed with 200 g of acetonitrile, and then dried in a vacuum dryer at 50°C for three hours. Five grams of the dried white powder was transferred to a 100 mL round-bottom flask and dissolved in 30 g of dimethylformamide. Then, 7.2 g of triethylamine and 6.7 g of di(N-succinimidyl)carbonate were added to the solution, and the mixture was stirred at room temperature overnight. To the reaction liquid, 1.75 g of succinic acid dihydrazide and 60 g of dimethyl sulfoxide were added, and the mixture was stirred at room temperature for three hours. Then, 0.1 g of succinic acid dihydrazide was added and the mixture was stirred for six days. Thereafter, the reaction liquid was added dropwise to 2 L of ethyl acetate to precipitate a solid. The obtained solid was washed with 50 g of acetone and dried in a vacuum dryer at 40°C for three hours to obtain 6.5 g of a compound of formula (A12). 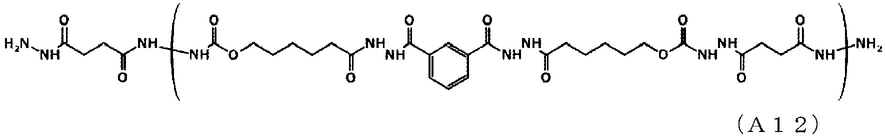 1H-NMR (DMSO, δ ppm) 1.28 to 1.39 (28H, m, CH₂), 1.49 to 1.62 (56H, m, CH₂), 2.11 to 2.23 (28H, m, O=CCH₂), 2.35 (32H, br, O=CCH₂), 4.00 (28H, br, OCH₂), 4.18 (4H, br, NH₂), 4.77 (2H, br, OH), 7.67 (7H, t, benzene ring), 8.01 (14H, d, benzene ring), 8.30 (7H, br, benzene ring), 8.95 (14H, br, NHNH), 9.04 (2H, br, NHNH), 9.66 (14H, br, NHNH), 9.81 (14H, br, NHNH), 10.37 (14H, br, NHNH)

Upon adding a small amount of a 12% sodium hypochlorite aqueous solution to the obtained compound of formula (A12), foaming occurred. 1H-NMR analysis confirmed the generation of succinic acid, 6-hydroxyhexanoic acid, and isophthalic acid as degradation products. An amount of 0.5 g of succinic acid and 0.5 g of 6-hydroxyhexanoic acid were each soluble in 10 mL of ion exchange water having a temperature of 20°C, exhibiting water solubility, while 0.3 g of isophthalic acid was insoluble in 10 mL of ion exchange water having a temperature of 20°C, exhibiting water insolubility.

An amount of 2.0 g of the compound of formula (A12), 2.0 g of dimethylformamide, and 0.15 g of resorcinol diglycidyl ether (EX-201-IM available from Nagase ChemteX Corporation) were mixed to prepare a composition for producing a degradable formed product. The composition was applied onto a glass substrate to give a cured film thickness of 10 µm, and heated at 80°C for three hours to obtain a degradable formed product. The degradable formed product was insoluble in ion exchange water, but when it was immersed in a 1% sodium hypochlorite aqueous solution having a temperature of 20°C, foaming occurred, and the degradable formed product was completely removed from the substrate within 15 minutes. 1H-NMR analysis confirmed the presence of succinic acid and 6-hydroxyhexanoic acid in the degradation products.

### (Example 13)

An amount of 3.30 g of 1,12-bis(2,5-dioxy-1-pyrrolidinyl) 2,5,8,11-tetraoxadodecanedioate, 26 g of dimethylformamide, and 1.6 g of triethylamine were mixed in a 100 mL round-bottom flask. Then, 0.88 g of succinic acid dihydrazide was added and the mixture was stirred at room temperature overnight. Next, 0.31 g of 4-hydroxybenzohydrazide was added and the mixture was further stirred at room temperature for six hours. To the reaction liquid, 37 g of ethyl acetate was added, and the mixture was left to stand at -10°C overnight to form a precipitate. The organic solvent was removed by decantation, and the precipitate was washed twice with 35 g of ethyl acetate. Lastly, the solvent was removed by evaporation to obtain a compound having a structure of formula (A13) as a white solid at a yield of 79%. 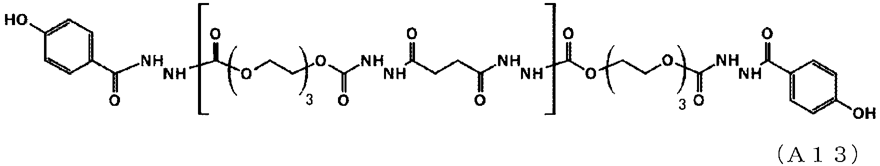 1H-NMR (DMSO, δ ppm) 2.34 (36H, br, CH₂CO), 3.54 (40H, br, CH₂O), 3.59 (40H, br, CH₂O), 4.10 (40H, br, CH₂OCO), 6.81 (4H, d, benzene ring), 7.72 (4H, d, benzene ring), 9.07 (20H, br, NHNH), 9.67 (20H, br, NHNH), 10.02 (2H, br, OH)

Upon adding a small amount of a 12% sodium hypochlorite aqueous solution to the obtained compound of formula (A13), foaming occurred. 1H-NMR analysis confirmed the generation of succinic acid and triethylene glycol as degradation products. An amount of 0.5 g of succinic acid and 2 g of triethylene glycol were each soluble in 10 mL of ion exchange water having a temperature of 20°C, exhibiting water solubility.

An amount of 1.0 g of the compound of formula (A13), 0.14 g of polyglycerol polyglycidyl ether (GEX-521 available from Nagase ChemteX Corporation), 0.06 g of benzyltriethylammonium chloride, 1 g of methanol, and 0.03 g of a leveling agent (BYK-348 available from BYK Japan KK) were added to 5 g of ion exchange water to prepare a composition for producing a degradable formed product. The composition was applied onto a glass petri dish to give a cured film thickness of 10 µm. The applied composition was dried and cured at 120°C for three hours to obtain a degradable cross-linked product. The degradable formed product was insoluble in ion exchange water, but when it was immersed in a 1% sodium hypochlorite aqueous solution having a temperature of 20°C, foaming occurred, and the degradable formed product was completely removed from the substrate within four minutes. Here, 1H-NMR analysis confirmed the presence of succinic acid and triethylene glycol in the degradation products.

### (Comparative Example 1)

An amount of 30 g of a bisphenol A epoxy resin (jER828 available from Mitsubishi Chemical Corporation), 0.12 g of tetramethylammonium chloride, and 28.5 g of ethylparaben were mixed in a 300 mL separable flask. The mixture was stirred at 100°C overnight and then brought to room temperature. To the resulting mixture, 93 g of chloroform and 30 g of ion exchange water were added, followed by a liquid separation process. The lower layer was returned to the flask and concentrated under reduced pressure at 75°C. To the residue, 42 g of methanol and 10.1 g of hydrazine monohydrate were added, and the mixture was stirred at 70°C for three hours. Thereafter, 20.2 g of hydrazine monohydrate was further added and the mixture was further stirred overnight. After the reaction liquid was concentrated under reduced pressure at 75°C, 47 g of dimethylformamide and 9.3 g of triethylamine were added and the mixture was cooled to 0°C. Then, 14.1 g of methacrylic anhydride was added dropwise and the mixture was stirred at room temperature for one hour. The reaction liquid was transferred to a 200 mL round-bottom flask and concentrated at 80°C with an evaporator. The concentrated liquid was added dropwise to 1 L of ion exchange water, and a white precipitate was collected. The precipitate was dried in a vacuum dryer at 40°C to obtain a compound of formula (B) at a yield of 81%. 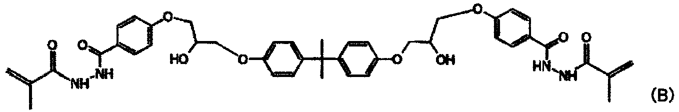 1H-NMR (DMSO, δ ppm) 1.57 (6H, S, CH₃CCH₃), 1.85 (6H, dd, C=CCH₃), 3.97 to 4.17 (10H, m, OCH₂CHCH₂O), 4.12 (4H, s, COOCH₂), 5.47 to 5.49 (2H, m, C=CH₂), 5.89 to 5.91 (2H, m, C=CH₂), 6.64 to 6.86 (4H, m, CH=CH), 6.98 to 7.01 (4H, m, CH=CH), 7.08 to 7.11 (4H, m, CH=CH), 7.78 to 7.81 (4H, m, CH=CH), 9.22 (2H, S, NHNH), 9.62 (2H, S, NHNH)

Upon adding a small amount of a 12% sodium hypochlorite aqueous solution to the obtained compound of formula (B), foaming occurred. 1H-NMR analysis confirmed the generation of a compound of formula (C) as a degradation product. An amount of 0.3 g of the compound of formula (C) was insoluble in 10 mL of ion exchange water having a temperature of 20°C, exhibiting water insolubility.

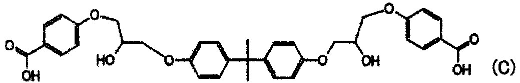

An amount of 0.3 g of the compound of formula (B), 0.1 g of acryloylmorpholine (ACMO available from KJ Chemicals Corporation), and 12 mg of a photopolymerization initiator (Omnirad 907 available from IGM Resins) were dissolved in 1 g of N-methylpyrrolidone to prepare a composition for producing a degradable formed product. The composition was applied onto a glass plate to give a dry film thickness of 10 µm. After drying the composition with a fan dryer at 150°C for 5 minutes, the dried composition was exposed to ultraviolet rays at 500 mJ/cm² using a UV irradiation device (Unicure UVH-1500M available from USHIO Inc.) to obtain a degradable formed product. The degradable formed product was insoluble in ion exchange water. When it was immersed in a 1% sodium hypochlorite aqueous solution having a temperature of 20°C, foaming occurred, but the coating film remained on the substrate even after 30 minutes.

### (Comparative Example 2)

An amount of 5.0 g of lactic acid hydrazide and 96 g of ion exchange water were mixed in a 200 mL round-bottom flask. After dropwise adding 1.44 g of concentrated sulfuric acid, the mixture was refluxed in an oil bath at 120°C for 15 hours. The reaction liquid was added dropwise to 224 g of ethanol, and a precipitated solid was removed by filtration. The filtrate was concentrated with an evaporator at 70°C to precipitate a white solid. The white solid was washed with 11 g of acetone to obtain a compound of formula (D) at a yield of 38%. 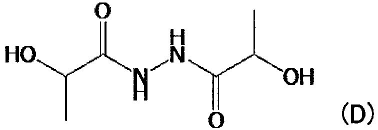 1H-NMR (DMSO, δ ppm) 1.24 (6H, d, CH₃), 4.04 to 4.11 (2H, m, CH), 5.44 (2H, dd, OH), 9.19 (2H, br, NHNH)

An amount of 1.5 g of polymeric diphenyl methane diisocyanate (Millionate MR-200 available from Tosoh Corporation) and 1.0 g of the compound of formula (D) were dissolved in 10 mL of dimethylformamide. To the solution, 75 mg of di-n-butyltin dilaurate was added and the mixture was stirred at 90°C for 30 minutes to prepare a composition for producing a degradable formed product. The composition was applied onto a glass plate to give a dry film thickness of 10 µm, and then cured with a fan dryer at 90°C for two hours.

The degradable formed product was insoluble in ion exchange water. When it was immersed in a 1% sodium hypochlorite aqueous solution having a temperature of 20°C, foaming occurred, but the coating film remained on the substrate even after 30 minutes.

### (Degradability evaluation)

The formed products with a film thickness of 10 µm prepared in Examples 1 to 13 and Comparative Examples 1 and 2 were each immersed in a 1% sodium hypochlorite aqueous solution. After 1, 2, 3, 4, 5, 10, 15, 20, and 30 minutes from the immersion, the presence of the remaining coating film was visually observed. Table 1 shows the results.

**[Table 1]**

| | Degradable cross-linking agent | Time taken to remove coating film |
|---|---|---|
| Example 1 | Formula (A1) | 10 minutes |
| Example 2 | Formula (A2) | 1 minute |
| Example 3 | Formula (A3) | 4 minutes |
| Example 4 | Formula (A4) | 2 minutes |
| Example 5 | Formula (A5) | 2 minutes |
| Example 6 | Formula (A6) | 10 minutes |
| Example 7 | Formula (A7) | 15 minutes |
| Example 8 | Formula (A8) | 20 minutes |
| Example 9 | Formula (A9) | 10 minutes |
| Example 10 | Formula (A10) | 10 minutes |
| Example 11 | Formula (A11) | 20 minutes |
| Example 12 | Formula (A12) | 15 minutes |
| Example 13 | Formula (A13) | 4 minutes |
| Comparative Example 1 | Formula (B) | Unremovable even after 30 minutes |
| Comparative Example 2 | Formula (D) | Unremovable even after 30 minutes |

The coating films of the formed products of Comparative Examples 1 and 2 could not be removed even after 30 minutes, while all the formed products of Examples 1 to 13 could be removed within 20 minutes. In particular, with regard to the formed products of Examples 1 to 6 and 13, as all the degradation products containing Z in formula (1) were soluble in water, these coating films were removed in a shorter time. Moreover, with regard to the formed products of Examples 7 to 12, as water-insoluble compounds were also generated as degradation products containing Z in formula (1), there was a tendency that the higher the molar ratio or molecular weight of the water-soluble degradation product, the shorter the time taken to remove the coating film. Further, with regard to the formed products of Examples 2, 4, 5, 7, 8, and 9, each using a degradable cross-linking agent with k in formula (1) being 1 or more, the coating films also tended to be removed in a short time.

Embodiment 1 of the present disclosure relates to a degradable cross-linking agent, including a compound represented by the following formula (1): 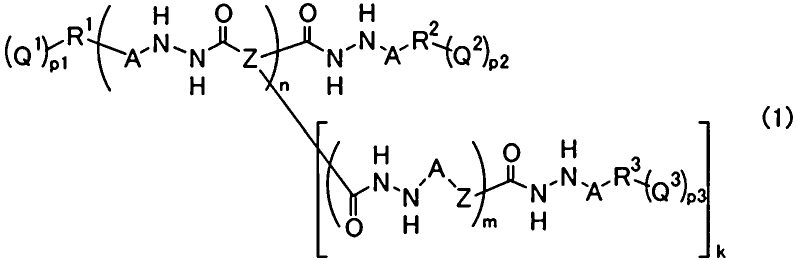 wherein
- n ≥ 1, k ≥ 0, m ≥ 0, p1 ≥ 1, p2 ≥ 1, and p3 ≥ 1;
- R¹, R², and R³ each independently represent a single bond or a C1-C500 hydrocarbon group optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- each A independently represents a carbonyl group or a single bond; and
- each Z independently represents a divalent or higher-valent group containing a siloxane structure or a C1-C500 hydrocarbon group optionally containing a heteroatom,
   the degradable cross-linking agent, when subjected to a reaction with an oxidizing agent after cross-linking, generating at least one water-soluble degradation product including a Z-containing carboxylic acid, a Z-containing alcohol, a Z-containing amine, or a Z-containing thiol and having a water solubility of 30 g/L or more.

Embodiment 2 of the present disclosure is the degradable cross-linking agent according to Embodiment 1,
wherein the water-soluble degradation product is represented by the following formula (2):

Y-Z(Y)ₖ-Y

wherein each Y independently represents a carboxyl group when bound to a carbon atom in Z, or a hydrogen atom when bound to an oxygen atom, a nitrogen atom, or a sulfur atom in Z; and Z and k are each as defined in formula (1).

Embodiment 3 of the present disclosure is the degradable cross-linking agent according to Embodiment 1 or 2,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

Embodiment 4 of the present disclosure is the degradable cross-linking agent according to any one of Embodiments 1 to 3,
wherein p1 = 1, p2 = 1, or p3 = 1.

Embodiment 5 of the present disclosure is the degradable cross-linking agent according to any one of Embodiments 1 to 4,
wherein k ≥ 1.

Embodiment 6 of the present disclosure is the degradable cross-linking agent according to any one of Embodiments 1 to 5,
wherein the degradable cross-linking agent contains, within its molecule, two or more Z groups that differ from each other in structure.

Embodiment 7 of the present disclosure is the degradable cross-linking agent according to any one of Embodiments 1 to 6,
wherein the degradable cross-linking agent, when subjected to the reaction with the oxidizing agent, further generates at least one poorly water-soluble degradation product including a Z-containing carboxylic acid, a Z-containing alcohol, a Z-containing amine, or a Z-containing thiol and having a water solubility of less than 30 g/L.

Embodiment 8 of the present disclosure is the degradable cross-linking agent according to Embodiment 7,
wherein the water-soluble degradation product has a larger average molecular weight than the poorly water-soluble degradation product.

Embodiment 9 of the present disclosure is the degradable cross-linking agent according to Embodiment 7 or 8,
wherein a total molar amount of the water-soluble degradation product generated by the reaction with the oxidizing agent is at least 1.3 times a total molar amount of the poorly water-soluble degradation product.

Embodiment 10 of the present disclosure is the degradable cross-linking agent according to any one of Embodiments 1 to 6,
wherein substantially all degradation products containing Z generated by the reaction with the oxidizing agent consist of the water-soluble degradation product.

Embodiment 11 of the present disclosure is the degradable cross-linking agent according to any one of Embodiments 1 to 10,
wherein Z contains a structure represented by the following formula (4):

-(R^{A}O)ₕ- (4)

wherein R^{A} represents a hydrocarbon group optionally having a C1-C5 substituent, and h represents 1 or more.

Embodiment 12 of the present disclosure is the degradable cross-linking agent according to any one of Embodiments 1 to 11,
wherein Z contains at least one hydrophilic functional group selected from the group consisting of a sulfonic acid, a sulfonate, a carboxylic acid, a carboxylate, a hydroxy group, and -NHR^{B} where R^{B} represents a hydrogen atom or a hydrocarbon group optionally having a C1-C5 substituent.

Embodiment 13 of the present disclosure relates to a composition, containing
the degradable cross-linking agent according to any one of Embodiments 1 to 12, and
at least one selected from the group consisting of a curable resin, a polymerization initiator, and a solvent.

Embodiment 14 of the present disclosure relates to a degradable cross-linked product, formed from the composition according to Embodiment 13.

Embodiment 15 of the present disclosure is the degradable cross-linked product according to Embodiment 14, which is degradable with an oxidizing agent.

Embodiment 16 of the present disclosure relates to a method for degrading a degradable cross-linked product, including
bringing the degradable cross-linked product according to Embodiment 14 or 15 into contact with an aqueous solution containing an oxidizing agent at 100°C or lower.

## Claims

1. A degradable cross-linking agent, comprising a compound represented by the following formula (1): 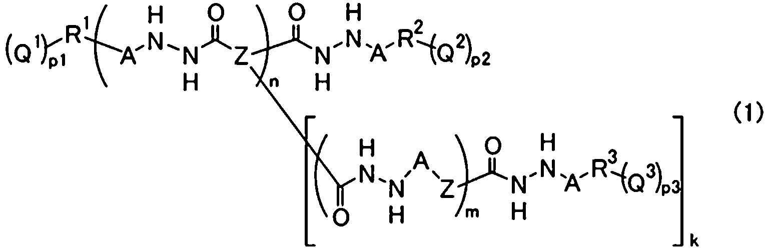 wherein
- n ≥ 1, k ≥ 0, m ≥ 0, p1 ≥ 1, p2 ≥ 1, and p3 ≥ 1;
- R¹, R², and R³ each independently represent a single bond or a C1-C500 hydrocarbon group optionally containing a substituent or a heteroatom;
- Q¹, Q², and Q³ each independently represent at least one reactive functional group selected from the group consisting of a hydroxy group, an amino group, a thiol group, a hydrazide group, a carboxylic acid group, an acid anhydride group, a vinyl group, an allyl group, an acrylate group, a methacrylate group, a crotonate group, an isoprenyl group, an acrylamide group, a methacrylamide group, a crotonamide group, an epoxy group, an oxetane group, an oxazoline group, an isocyanate group, a carbodiimide group, a methylol group, a silanol group, a hydroxysilyl group, and an alkoxysilyl group;
- each A independently represents a carbonyl group or a single bond; and
- each Z independently represents a divalent or higher-valent group containing a siloxane structure or a C1-C500 hydrocarbon group optionally containing a heteroatom,
the degradable cross-linking agent, when subjected to a reaction with an oxidizing agent after cross-linking, generating at least one water-soluble degradation product comprising a Z-containing carboxylic acid, a Z-containing alcohol, a Z-containing amine, or a Z-containing thiol and having a water solubility of 30 g/L or more.

2. The degradable cross-linking agent according to claim 1,
wherein the water-soluble degradation product is represented by the following formula (2):
Y-Z(Y)ₖ-Y
wherein each Y independently represents a carboxyl group when bound to a carbon atom in Z, or a hydrogen atom when bound to an oxygen atom, a nitrogen atom, or a sulfur atom in Z; and Z and k are each as defined in formula (1).

3. The degradable cross-linking agent according to claim 1 or 2,
wherein p1 ≥ 2, p2 ≥ 2, or p3 ≥ 2, and
the plurality of reactive functional groups as Q¹ are different from each other, or the plurality of reactive functional groups as Q² are different from each other, or the plurality of reactive functional groups as Q³ are different from each other.

4. The degradable cross-linking agent according to claim 1 or 2,
wherein p1 = 1, p2 = 1, or p3 = 1.

5. The degradable cross-linking agent according to any one of claims 1 to 4,
wherein k ≥ 1.

6. The degradable cross-linking agent according to any one of claims 1 to 5,
wherein the degradable cross-linking agent contains, within its molecule, two or more Z groups that differ from each other in structure.

7. The degradable cross-linking agent according to any one of claims 1 to 6,
wherein the degradable cross-linking agent, when subjected to the reaction with the oxidizing agent, further generates at least one poorly water-soluble degradation product comprising a Z-containing carboxylic acid, a Z-containing alcohol, a Z-containing amine, or a Z-containing thiol and having a water solubility of less than 30 g/L.

8. The degradable cross-linking agent according to claim 7,
wherein the water-soluble degradation product has a larger average molecular weight than the poorly water-soluble degradation product.

9. The degradable cross-linking agent according to claim 7 or 8,
wherein a total molar amount of the water-soluble degradation product generated by the reaction with the oxidizing agent is at least 1.3 times a total molar amount of the poorly water-soluble degradation product.

10. The degradable cross-linking agent according to any one of claims 1 to 9,
wherein substantially all degradation products containing Z generated by the reaction with the oxidizing agent consist of the water-soluble degradation product.

11. The degradable cross-linking agent according to any one of claims 1 to 10,
wherein Z contains a structure represented by the following formula (4):
-(R^{A}O)ₕ- (4)
wherein R^{A} represents a hydrocarbon optionally having a C1-C5 substituent, and h represents 1 or more.

12. The degradable cross-linking agent according to any one of claims 1 to 11,
wherein Z contains at least one hydrophilic functional group selected from the group consisting of a sulfonic acid, a sulfonate, a carboxylic acid, a carboxylate, a hydroxy group, and -NHR^{B} where R^{B} represents a hydrogen atom or a hydrocarbon optionally having a C1-C5 substituent.

13. A composition, comprising
the degradable cross-linking agent according to any one of claims 1 to 12, and
at least one selected from the group consisting of a curable resin, a polymerization initiator, and a solvent.

14. A degradable cross-linked product, formed from the composition according to claim 13.

15. The degradable cross-linked product according to claim 14, which is degradable with an oxidizing agent.

16. A method for degrading a degradable cross-linked product, comprising
bringing the degradable cross-linked product according to claim 14 or 15 into contact with an aqueous solution containing an oxidizing agent at 100°C or lower.
